# EUROPEAN PATENT APPLICATION

(11) **EP 1 946 795 A1**
(43) Date of publication of application: **23.07.2008**
(21) Application number: 07106109.7
(22) Date of filing: 13.04.2007
(51) Int. Cl.: A61N 5/10

(54) **Radiation irradiation method and radiotherapy apparatus controller**

(30) Priority: 16.01.2007 JP 2007007332
(71) Applicant: MITSUBISHI HEAVY INDUSTRIES, LTD., Tokyo 108-8215 (JP)
(72) Inventor: Urano, Susumu c/o MITSUBISHI HEAVY INDUSTRIES, LTD., Hiroshima (JP); Kaneko, Shuji c/o MITSUBISHI HEAVY INDUSTRIES, LTD., Hiroshima (JP); Takahashi, Kunio c/o MITSUBISHI HEAVY INDUSTRIES, LTD., Hiroshima (JP); Tsukuda, Kazuhiro c/o MITSUBISHI HEAVY INDUSTRIES, LTD., Hiroshima (JP)
(74) Representative: Bongiovanni, Simone

(57) **Abstract**

A radiation irradiation method includes the steps (a) and (b). The step (a) is the step of irradiating a first part of a subject with first radiation radiated in a radial pattern from a first point. The step (b) is the step of irradiating a second part different from said first part of said subject with second radiation radiated in a radial pattern from a second point. A first position of said first point relative to said subject correspond to a second position of said second point relative to said subject.

## Description

### Background of the Invention

### 1. Field of the Invention

The present invention relates to a radiation irradiation method and a radiotherapy apparatus controller, and especially relates to a radiation irradiation method and a radiotherapy apparatus controller that are used for irradiating an affected area with radiation for radiotherapy.

### 2. Description of the Related Art

The radiotherapy is known which is treatment of disease by irradiating an affected area (a tumor) with therapeutic radiation. Radiation produced by the bremsstrahlung is exemplified as the therapeutic radiation. It is desired for the radiotherapy that effectiveness of treatment is high and that a radiation dose with which normal cells are irradiated is smaller than a radiation dose with which cells in the affected area are irradiated. That is to say, it is desired for the radiotherapy to control an area irradiated with the therapeutic radiation with higher precision than ever and to control the radiation dose of the therapeutic radiation with which the affected area is irradiated with higher precision than ever.

In Japanese Laid-Open Patent Application JP-Heisei 8-511452T (corresponding to W09428971, EP0810006, EP0810005, EP0703805), a radiotherapy apparatus is disclosed which has a degree of freedom of regulated angle for radiating beams only in a plane of a gantry. It is not necessarily for a radiation shield to be fixed, to be attached to the gantry, or to rotate with consistently shielding against a main beam. A regulated movement reduces a danger of a collision between a patient and the gantry, and provides an extremely accurate radiotherapy planning. The radiotherapy apparatus composed as described above can include an X-ray tomography image-formation system into one gantry. Two systems cooperate, and design and execute a treatment activity in which a treatment area with non-uniform figure is accurately irradiated and a tissue surrounding the area is irradiated at minimum by using many of configuration parts of the same hardware. The radiotherapy apparatus can include a collimator for minimizing an irradiation to benign tumor neighboring malignant tumor by changing a width of a radiation fan beam when the treatment area of the patient intersects an area exposed to the beam. The width of the fan beam can be controlled so that multiple contiguity resemblance slices can be treated for a certain period in a treatment period.

In Japanese Patent JP3746744, a radiotherapy apparatus is disclosed which has superior treatment performance. The radiotherapy apparatus includes: an irradiation head including a therapeutic radiation generation part which has an electron gun, a linear accelerator and a target and a gimbals mechanism which oscillates the therapeutic radiation generation part; a supporting and moving mechanism supporting and moving the irradiation head on a predetermined spherical coordinates; a microwave generation part allocated on a static position for generating microwave to be supplied to above mentioned irradiation head; and a waveguide part whose one end is electromagnetically connected to the microwave generation part and whose other end is electromagnetically connected to the linear accelerator. The radiotherapy apparatus is characterized by connecting a waveguide of the waveguide part mounted on above mentioned gimbal mechanism with a waveguide of the waveguide part from the micro wave generation part by using a flexible waveguide.

In Japanese Patent JP3746747 (Japanese Laid-Open Patent Application JP2004-097646), a radiotherapy apparatus is disclosed which can monitor a condition of a treatment field in real time even during a radiation irradiation treatment. The radiotherapy apparatus includes: a radiation irradiation head for irradiating a treatment field of a subject with therapeutic radiation; a X-ray source for irradiating the treatment field of the subject with diagnostic X-ray; and a sensor array for detecting transmission X-ray of the diagnostic X-ray that penetrated the subject under the treatment and outputting it as diagnostic image data. The sensor array moves in conjunction with movement of the radiation irradiation head.

In Japanese Laid-Open Patent Application JP2002-065876, a radiotherapy method is disclosed which prevents parts that are excessively irradiated with overlapping radiation dose on a border area of irradiation sections and parts that are irradiated with no radiation dose, even in a case that fractionated irradiation is carried out by dividing the irradiation section into a plurality of sections in direction of predetermined width of an irradiation field because the whole tumor cannot be irradiated with one time radiation. The radiotherapy method is a treatment method including steps of: dividing the irradiation section into several parts in direction parallel to the width of the irradiation field in predetermined direction by collimating means; and irradiating a treated area in an affected area with radiation in units of respective irradiation sections. When the border area on which the divided irradiation sections face each other, the collimating means is moved in direction parallel to the width of the irradiation field in order to reduce radiation dose towards the side of the facing irradiation section.

In Japanese Laid-Open Patent Application JP2004-321408 (corresponding to US6984835), a radiation irradiation apparatus is disclosed which has a large irradiation field and secures a flatness of a distribution of radiation dose in the irradiation field without strengthening a performance of an accelerator and an irradiation field enlargement device. The radiation irradiation apparatus irradiates an irradiated area set on an irradiation table with radiation beam transferred from an accelerator. The radiation irradiation apparatus is characterized by including: a beam blocking means for blocking the radiation beam; a position control means for controlling the position of the irradiation table so that the whole irradiated area is irradiated in a plurality of irradiation areas including superposition areas specified by a plurality of irradiation using the radiation beam; and a multi-leaf collimator control means for leveling a distribution of radiation dose to the whole irradiated area including the superposition area by making a distribution of the radiation dose in the superposition area of the respective irradiation areas have a gradient and irradiating with the plurality of radiation beam.

International publication WO03018131 (corresponding to US7085347, US6977987) discloses a radiological treatment apparatus. The radiological treatment apparatus includes a radiation head having a linear accelerator and a head waveguide part having one end part electromagnetically connected to the linear accelerator; a supportedly moving mechanism movably supporting the radiation head on a predetermined first spherical coordinate; a microwave oscillator generating microwave supplied to the radiation head and disposed at a stationary position; a fixed waveguide part having one end part electromagnetically connected to the microwave oscillator and the other end part positioned on the supportedly moving mechanism; and a movable waveguide electromagnetically connected to the other end part of the fixed waveguide part having one end part positioned on the supportedly moving mechanism.

U. S. patent US5,442,675 discloses a dynamic collimator for radiation therapy. The radiation therapy machine having a radiation source for directing a beam of radiation along a beam plane toward a patient with a treatment volume, the therapy machine including a collimator disposed between the radiation source and the patient, to control the beam width normal to the beam plane, the machine including a means for supporting and moving the patient with respect to the beam plane along a translation axis wherein the treatment volume includes a plurality of adjacent slices, each slice associated with a sinogram, indicating desired fluence profiles to be directed toward one associated slice, the machine includes: a comparison means for comparing sinograms of adjacent slices to generate a difference value; a control signal means receiving the difference value from the comparison means for indicating whether the difference value is within a predetermined limit by means of a correlation signal; and a collimator control means receiving the correlation signal from the control signal means for controlling the collimator to adjust the beam width to simultaneously irradiate adjacent slices of the treatment volume when the correlation signal indicates that the difference value is within the predetermined limit.

U. S. patent US5, 548, 627 discloses a radiation therapy machine for treating a patient having a treatment volume. The machine includes: a gantry for rotation with a gantry plane; a radiation source disposed on the gantry for rotation with the gantry about an axis of rotation, the radiation source directing a radiation beam of a predetermined intensity appropriate to treat tumorous tissue toward the axis of rotation form a plurality of first gantry angles along the gantry plane; an x-ray source disposed on the gantry for rotation with the gantry about the axis of rotation, the x-ray source for producing an x-ray beam directed toward the axis of rotation from a plurality of second gantry angles in a plane parallel to the gantry plane, the radiation source and the x-ray source positioned in separate parallel planes so that the radiation beam and x-ray beam do not intersect; an x-ray detector disposed on the gantry and diametrically opposed to the x-ray source for receiving the x-ray beams therefrom and producing x-ray data; and a computer means for receiving the x-ray data and generating a tomographic image therefrom.

U. S. patent US5, 724, 400 discloses a radiation therapy machine for treating a patient with high energy radiation. The radiation therapy machine includes: a gantry for rotation within a gantry plane; a table disposed along an axis of translation for supporting a patient and for moving the patient along the axis of translation, the axis of translation positioned within the rotation of the gantry; a radiation source disposed on the gantry for producing a radiation beam within a fan beam plane substantially parallel to the gantry plane, the beam including a plurality of rays diverging in the beam plane about one central ray, the central ray directed at the patient from a variety of gantry angles along the gantry plane; and an attenuation means disposed between the radiation source and the patient for independently controlling the intensity of each ray as a function of gantry angle. The attenuation means includes: a plurality of radiation attenuating leaves; a supporting member positioned generally between the radiation source and the patient for guiding the leaves between a closed state within the radiation beam, each leaf thus occluding one ray of the beam, and an open state outside of the radiation beam to allow unobstructed passage of the ray; motivation means for independently moving each leaf between the open and closed states; and timing means communicating with the motivation means for controlling the ratio of the period of time during which each leaf is in the closed state to the period during which each leaf is in the open state to control the average energy fluence of each ray of the beam.

### Summary of the Invention

Therefore, an object of the present invention is to provide a radiation irradiation method and a radiotherapy apparatus controller for controlling a position of an area which is irradiated with the therapeutic radiation in a subject with higher precision than ever and controlling the radiation dose with which the area is irradiated with higher precision than ever.

Another object of the present invention is to provide a radiation irradiation method and a radiotherapy apparatus controller for controlling a radiation dose with which the area of the subject is irradiated with higher precision than ever and for miniaturizing a radiation irradiation device that emits the radiation.

Still another object of the present invention is to provide a radiation irradiation method and a radiotherapy apparatus controller for controlling a radiation dose with which the area of the subject is irradiated with higher precision than ever and for miniaturizing an irradiation field shape controller that changes a shape of an irradiation field which is irradiated with the radiation.

This and other objects, features and advantages of the present invention will be readily ascertained by referring to the following description and drawings.

In order to achieve an aspect of the present invention, the present invention provides a radiation irradiation method including: (a) irradiating a first part of a subject with first radiation radiated in a radial pattern from a first point; and (b) irradiating a second part different from the first part of the subject with second radiation radiated in a radial pattern from a second point. A first position of the first point relative to the subject correspond to a second position of the second point relative to the subject.

In the radiation irradiation method, the step (a) may include (a1) irradiating the first part with the first radiation radiated by a radiation irradiation apparatus which radiates radiation in a radial pattern from a point. The step (b) may include (b1) moving the radiation irradiation apparatus after the step (a1) such that the radiation irradiation apparatus radiates the second radiation.

The radiation irradiation method may further includes (c) changing a shape of irradiation field irradiated with the radiation in the subject by an irradiation field shape controlling device which shields against a part of the radiation.

In the radiation irradiation method, the step (a) may include (a1) irradiating the first part with the first radiation radiated by a radiation irradiation apparatus which radiates radiation in a radial pattern from a point. The step (b) may include (b1) moving the radiation irradiation apparatus after the step (a1) such that the radiation irradiation apparatus radiates the second radiation and the point does not move relatively to the subject, and (b2) irradiating a third part of the subject with third radiation radiated by the radiation irradiation apparatus while the radiation irradiation apparatus is moving.

The radiation irradiation method may further include (c) changing a shape of irradiation field irradiated with the radiation in the subject by an irradiation field shape controlling device which shields against a part of the radiation.

The radiation irradiation method may further include: (d) detecting a motion of the subject; and (e) moving a radiation irradiation apparatus which radiates radiation in a radial pattern from a point based on the motion such that the radiation irradiation apparatus radiates the first radiation to the first part and the second radiation to the second part even though the first part and the second part move.

In order to achieve another aspect of the present invention, the present invention provides computer program product with program code means for carrying out all steps according to any of the above-mentioned radiation irradiation methods if the program runs on a computer.

The present invention provides computer program product with program code means according to the above-mentioned computer program product which are stored on a storage means which can be read by the computer.

In order to achieve another aspect of the present invention, the present invention provides a radiotherapy apparatus controller including: an irradiation portion configured to irradiate a part of a subject with radiation by a radiation irradiation apparatus which radiates the radiation in a radial pattern from a point; and a head swing portion configured to move the radiation irradiation apparatus by a driving apparatus which moves the radiation irradiation apparatus relatively to the subject such that the point does not move relatively to the subject.

The radiotherapy apparatus controller may further include: a motion collecting portion configured to collect a motion of the subject by a motion detecting apparatus which detects the motion; and a moving body tracking portion configured to move the radiation irradiation apparatus based on the motion by the driving apparatus such that the radiation irradiation apparatus radiates the radiation to the part even though the part moves.

The radiotherapy apparatus controller may further include: a irradiation field shape controller configured to change a shape of irradiation field irradiated with the radiation in the subject by an irradiation field shape controlling device which shields against a part of the radiation.

The radiotherapy apparatus controller may further include: a motion collecting portion configured to collect a motion of the subject by a motion detecting apparatus which detects the motion; and a moving body tracking portion configured to move the radiation irradiation apparatus based on the motion by the driving apparatus such that the radiation irradiation apparatus radiates the radiation to the part even though the part moves.

In order to achieve another aspect of the present invention, the present invention provides a radiotherapy apparatus including a support member configured to be movable to a subject; a radiation irradiation apparatus configured to radiate radiation from a point; and a head swing mechanism configured to support the radiation irradiation apparatus movable to the support member such that the point is fixed to the support member.

In the radiotherapy apparatus, the head swing mechanism may support the radiation irradiation apparatus to the support member such that the radiation irradiation apparatus is rotatable to rotational axes.

In the radiotherapy apparatus, the head swing mechanism may include: an intermediate member which is supported by the support member such that the intermediate member is rotatable centering around a first axis. The radiation irradiation apparatus is supported by the intermediate member such that the radiation irradiation apparatus is rotatable centering around a second axis. The first axis intersects with the second axis.

In order to achieve another aspect of the present invention, the present invention provides a radiotherapy system including: a radiation irradiation apparatus configured to radiate radiation in a radial pattern from a point; a driving apparatus configured to move the radiation irradiation apparatus relatively to a subject; and a radiotherapy apparatus controller according to any of the above-mentioned radiotherapy apparatus controllers, configured to control the radiation irradiation apparatus and the driving apparatus.

### Brief Description of the Drawings

Fig. 1 is a block diagram showing an embodiment of a radiotherapy system according to the present invention;
Fig. 2 is a perspective view showing a radiotherapy apparatus;
Fig. 3 is a sectional view showing a therapeutic radiation irradiation device;
Fig. 4 is a perspective view showing an head swing mechanism;
Fig. 5 is a block diagram showing an embodiment of a radiotherapy apparatus controller according to the present invention;
Fig. 6 is a plain view showing an affected area of a patient seen from a hypothetical point source;
Fig. 7 is a sectional view showing therapeutic radiation emitted to a plurality of parts in the affected area;
Fig. 8 is a graph showing an intensity distribution of the therapeutic radiation in width direction;
Fig. 9 is a graph showing a dose distribution of the therapeutic radiation emitted to the affected area; and
Fig. 10 is a sectional view showing a comparative example of the therapeutic radiation emitted to the plurality of parts in the affected area.

### Description of the Preferred Embodiments

The invention will be now described herein with reference to illustrative embodiments. Those skilled in the art will recognize that many alternative embodiments can be accomplished using the teachings of the present invention and that the invention is not limited to the embodiments illustrated for explanatory purposed.

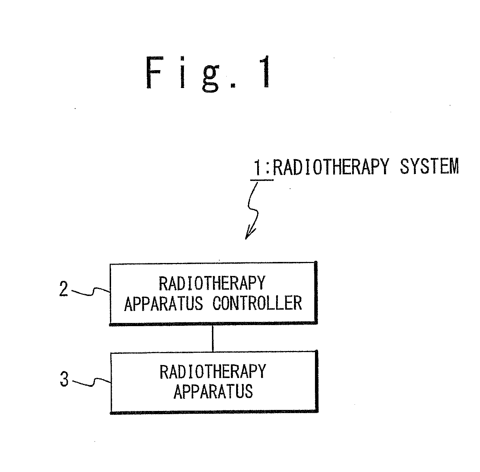

An embodiment of a radiotherapy system according to the present invention will be described referring to drawings. The radiotherapy system 1 includes a radiotherapy apparatus controller 2 and a radiotherapy apparatus 3 as shown in Fig. 1. The radiotherapy apparatus controller 2 is a computer such as a personal computer. The radiotherapy apparatus controller 2 is connected to the radiotherapy apparatus 3 so that information can be interactively transmitted between the radiotherapy apparatus controller 2 and the radiotherapy apparatus 3.

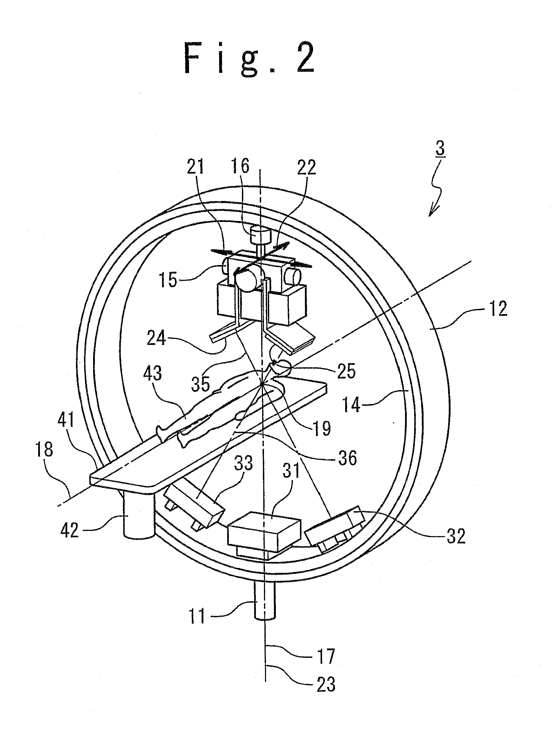

Fig. 2 shows the radiotherapy apparatus 3. The radiotherapy apparatus 3 includes a rotation drive device 11, an O-ring 12, a traveling gantry 14, a head swing mechanism 15, and a therapeutic radiation irradiation device 16. The rotation drive device 11 rotatably supports the O-ring 12 on a base centering around a rotational axis 17, rotates the O-ring 12 centering around the rotational axis 17 under the control of the radiotherapy apparatus controller 2, and outputs a rotational angle of the O-ring 12 against the base. The rotational axis 17 is parallel to the vertical direction. The O-ring 12 is formed in a ring shape centering around a rotational axis 18, and rotatably supports the traveling gantry 14 centering around the rotational axis 18. The rotational axis 18 is perpendicular to the vertical direction, and runs through an isocenter 19 included in the rotational axis 17. The rotational axis 18 is further secured to the O-ring 12, and, for this reason, rotates with the O-ring 12 centering around the rotational axis 17. The traveling gantry 14 is formed in ring shape centering around the rotational axis 18, and is arranged so as to be a concentric circle with the ring of the O-ring 12. The radiotherapy apparatus 3 further includes a traveling driving device not shown in Fig. 2. The traveling driving device rotates the traveling gantry 14 centering around the rotational axis 18 under control of the radiotherapy apparatus controller 2, and outputs a traveling angle of the traveling gantry 14 against the O-ring 12.

The head swing mechanism 15 is secured inside the ring of the traveling gantry 14, and supports the therapeutic radiation irradiation device 16 on the traveling gantry 14 so that the therapeutic radiation irradiation device 16 can be arranged inside the traveling gantry 14. The head swing mechanism 15 has a pan axis 21 and a tilt axis 22. The tilt axis 22 is secured to the traveling gantry 14, and is parallel to the rotational axis 18 without intersecting with the rotational axis 18. The pan axis 21 is orthogonal to the tilt axis 22. The head swing mechanism 15 rotates the therapeutic radiation irradiation device 16 centering around the pan axis 21 under the control by the radiotherapy apparatus controller 2, and rotates the therapeutic radiation irradiation device 16 centering around the tilt axis 22.

The therapeutic radiation irradiation device 16 radiates a therapeutic radiation 23 under the control of the radiotherapy apparatus controller 2. The therapeutic radiation 23 is radiated almost along a straight line running on the intersection where the pan axis 21 and the tilt axis 22 intersect each other. Since a part of the therapeutic radiation 23 is blocked, a shape of its irradiation field is controlled when the therapeutic radiation 23 is radiated to a patient. In addition, the therapeutic radiation 23 is formed so as to have a uniform distribution of intensity in the irradiation field.

Since the therapeutic radiation irradiation device 16 is supported by the traveling gantry 14, the therapeutic radiation 23 always and almost passes the isocenter 19 when the therapeutic radiation irradiation device 16 is once adjusted so as to face the isocenter 19 by the head swing mechanism 15, even when the O-ring 12 is rotated by the rotation drive device 11 or the traveling gantry 14 is rotated by the traveling driving device. That is to say, the therapeutic radiation 23 can be radiated to the isocenter 19 from an arbitrary direction through the traveling and the rotating.

The radiotherapy apparatus 3 further includes a plurality of imager systems. Concretely, the radiotherapy apparatus 3 includes diagnostic x-ray sources 24 and 25 and sensor arrays 32 and 33. The diagnostic X-ray source 24 is supported by the traveling gantry 14. The diagnostic X-ray source 24 is arranged inside the ring of the traveling gantry 14, and arranged at a position where an angle formed by a line segment connecting the isocenter 19 with the diagnostic X-ray source 24 and a line segment connecting the isocenter 19 with the therapeutic radiation irradiation device 16 is an acute angle. The diagnostic X-ray source 24 radiates a diagnostic X-ray 35 to the isocenter 19 under the control by the radiotherapy apparatus controller 2. The diagnostic X-ray 35 is a conical corn beam that is radiated from one point included in the diagnostic X-ray source 24 and whose cone point is the one point. The diagnostic X-ray source 25 is supported by the traveling gantry 14. The diagnostic X-ray source 25 is arranged inside the ring of the traveling gantry 14, and arranged at a position where an angle formed by a line segment connecting the isocenter 19 with the diagnostic X-ray source 25 and a line segment connecting the isocenter 19 with the therapeutic radiation irradiation device 16 is an acute angle. The diagnostic X-ray source 25 radiates a diagnostic X-ray 36 to the isocenter 19 under the control of the radiotherapy apparatus controller 2. The diagnostic X-ray 36 is a conical corn beam that is radiated from one point of the diagnostic X-ray source 25 and whose cone point is the one point.

The sensor array 32 is supported by the traveling gantry 14. The sensor array 32 receives the diagnostic X-ray 35 that is radiated by the diagnostic X-ray source 24 and transmits a subject around the isocenter 19, and produces a transfer image of the subject. The sensor array 33 is supported by the traveling gantry 14. The sensor array 33 receives the diagnostic X-ray 36 that is radiated by the diagnostic X-ray source 25 and transmits a subject around the isocenter 19, and produces a transfer image of the subject. Each of the sensor arrays 32 and 33 is exemplified in a FPD (Flat Panel Detector) or an X-ray II

### (Image Intensifier).

The radiotherapy apparatus 3 further includes a sensor array 31. The sensor array 31 is arranged so that a line segment connecting the sensor array 31 with the therapeutic radiation irradiation device 16 runs on the isocenter 19, and is secured inside the ring of the traveling gantry 14. The sensor array 31 receives the diagnostic x-ray 23 that is radiated by the therapeutic radiation irradiation device 16 and transmits a subject around the isocenter 19, and produces a transfer image of the subject. The sensor array 31 is exemplified in a FPD (Flat Panel Detector) or an X-ray II (Image Intensifier).

According to such imager systems, a transfer image centered around the isocenter 19 can be produced on the basis of image signals obtained by the sensor arrays 31, 32, and 33.

The radiotherapy apparatus 3 further include a couch 41 and a couch driving device 42. The couch 41 is used when a patient 43 to be treated by the radiotherapy system 1 lies down. The couch 41 includes holding fixtures that are not shown in Fig. 2. The holding fixtures keep the patient from moving, and fix the patient to the couch 41. The couch driving device 42 supports the couch 41 on a base, and moves the couch 41 under the control by the radiotherapy apparatus controller 2.

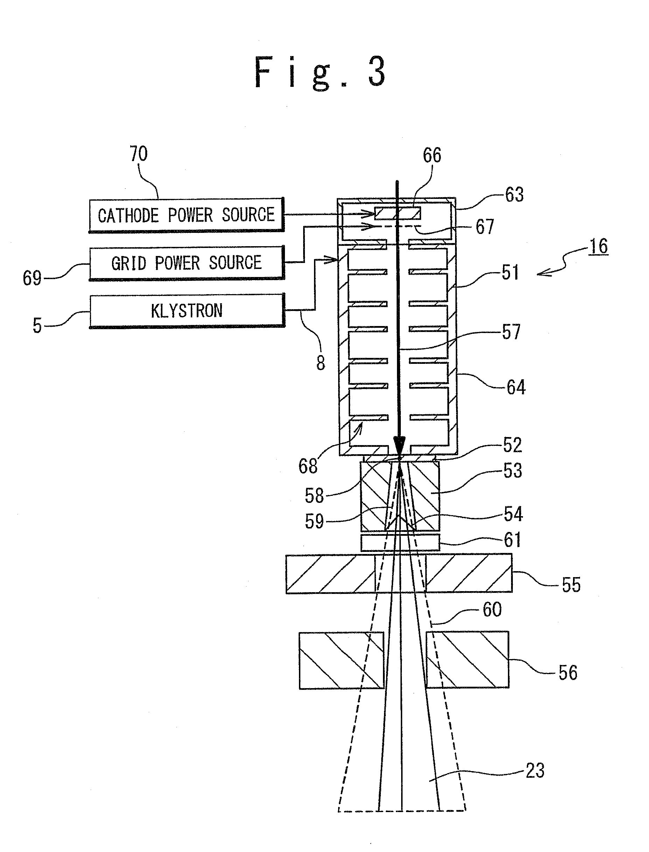

Fig. 3 shows the therapeutic radiation irradiation device 16. The therapeutic radiation irradiation device 16 includes an electron beam accelerator 51, an X-ray target 52, a primary collimator 53, a flattening filter 54, a dosimeter 61, a secondary collimator 55, and a multi-leaf collimator 56. The electron beam accelerator 51 emits an electron beam 57 generated by accelerated electrons to the X-ray target 52. The X-ray target 52 is composed of a material of higher atomic number (tungsten, tungsten alloy, and so on), and radiates a radiation 59 produced by the bremsstrahlung in emission of the electron beam 57. A radiation point of the radiation 59 has a constant distribution (area) depending on energy and property of the electron beam 57 emitted to the X-ray target 52. However, dimensions of the area are much smaller than the distance from the X-ray target 52 to the isocenter 19. Therefore, the position of radiating the radiation 59 can be treated as a point source for convenience. Hereinafter, this point source is referred to as a hypothetical point radiation source 58. The radiation 59 is radiated almost along a straight line running on the hypothetical point radiation source 58 that the X-ray target 52 includes internally. That is to say, the radiation 59 is a conical corn beam that is radiated from hypothetical point radiation source 58 in a radial pattern and whose cone point is the hypothetical point radiation source 58. The primary collimator 53 is composed of a material of higher atomic number (lead, tungsten, and so on), and shields against the radiation 59 so that an area other than the desired area is not irradiated with the radiation 59. The flattening filter 54 is, for example, composed of aluminum, and is formed in a plate having an approximately conical projection. The flattening filter 54 is arranged so that its projection faces the X-ray target side. A shape of the flattening filter is formed so that, after passing the flattening filter, dose in predetermined area of a plane perpendicular to its radiation direction is distributed almost uniformly. The secondary collimator 55 is composed of a material of higher atomic number (lead, tungsten, and so on), and shields against the radiation 60 so that an area other than the desired area is not irradiated with the radiation 60. The multi-leaf collimator 56 has a plurality of leaves for shielding against a part of the radiation 60. Each of the plurality of leaves is arranged along a plane including the hypothetical point radiation source 58, and is supported along the plane in a movable condition. The multi-leaf collimator 56 moves each of the plurality of leaves to arbitrary position under the control of radiotherapy apparatus controller 2. That is to say, when the therapeutic radiation 23 is radiated to the patient, the multi-leaf collimator 56 controls the shape of the irradiation field by shielding against a part of the radiation 60 under the control of the radiotherapy apparatus controller 2. In addition, the multi-leaf collimator 56 can be replaced by an irradiation field shape controller for controlling a shape of an irradiation field without equipping the plurality of leaves. Both may be called the irradiation field shape controlling device.

The dosimeter 61 is a transmission ionization chamber for measuring intensity of penetrating radiation, and is arranged in a position between the primary collimator 53 and the secondary collimator 55 so that the radiation 60 penetrates. The dosimeter 61 measures dose of the penetrating radiation 60, and outputs the dose to the radiotherapy apparatus controller 2. Such dosimeter 61 is preferable because non-destructive examination can be achieved. Other X-ray intensity detector different from the transmission ionization chamber can be used as the dosimeter 61. The X-ray intensity detector is exemplified in a semiconductor detector or a scintillation detector. Since it is preferable to arrange the semiconductor detector or the scintillation detector out of a radiation path because it is difficult to arrange them on the path as an alternative for the transmission ionization chamber. The semiconductor detector or the scintillation detector is, for example, secured on the traveling gantry 14 so as to be arranged in a position facing the therapeutic radiation irradiation device 16 at a distance from the isocenter 19. The ionization chamber is inferior in a responsibility because its time constant is a few seconds. The semiconductor detector and the scintillation detector have disadvantages that signal intensity is lower than that of the ionization chamber when being arranged out of the radiation path. However, they are preferable to the ionization chamber because a responsibility is improved.

The electron beam accelerating device 51 includes an electron beam generator 63 and an acceleration tube 64. The electron beam generator 63 includes a cathode 66 and a grid 67 . The acceleration tube 64 is formed in cylindrical shape, and includes a plurality of electrodes 68 lining up at appropriate intervals inside the cylinder. The radiotherapy apparatus 3 further includes a cathode power source 70 and a grid power source 69. The cathode power source 70 supplies, under the control of the radiotherapy apparatus controller 2, electric power to the cathode 66 so that the predetermined amount of electrons are emitted from the cathode 66 by heating the cathode 66 (that is to say, so that a predetermined temperature of the cathode 66 is maintained). The grid power source 69 supplies, under the control of the radiotherapy apparatus controller 2, voltage between the grid 67 and the cathode 66 so that the predetermined amount of electrons are emitted from the electron beam generator 63. A klystron 5 is connected to the acceleration tube 64 via a waveguide tube 8. The klystron 5 emits, under the control of the radiotherapy apparatus controller 2, a micro wave to the acceleration tube 64 via the waveguide tube 8 so that the acceleration tube 64 accelerates electrons emitted from the electron beam generator 63 such that the electrons have predetermined energy.

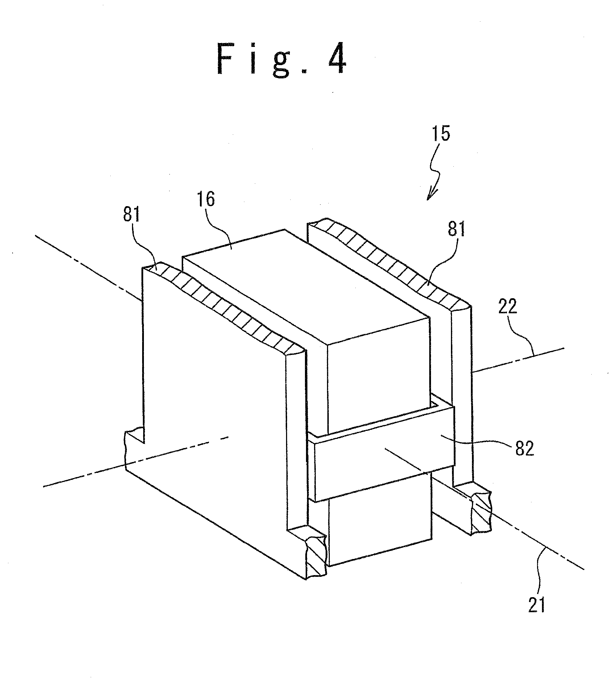

Fig. 4 shows the head swing mechanism 15. The head swing mechanism 15 includes a radiation device support member 81 and an intermediate member 82. The radiation device support member 81 is supported by the traveling gantry 14, and moves with the traveling gantry 14. The tilt axis 22 is fixed to the radiation device support member 81. The intermediate member 82 is rotatably supported by the radiation device support member 81 centering around the tilt axis 22. Since the intermediate member 82 contacts a part of the radiation device support member 81 when rotating centering around the tilt axis 22, a range of the rotation is limited. The pan axis 21 is fixed to the intermediate member 82. The therapeutic radiation irradiation device 16 is rotatably supported by the intermediate member 82 centering around the pan axis 21. Since the therapeutic radiation irradiation device 16 contacts a part of the intermediate member 82 when rotating centering around the pan axis 21, a range of the rotation is limited.

The head swing mechanism 15 further includes a pan axis driving device and a tilt axis driving device that are not shown in Fig. 4. The pan axis driving device rotates the therapeutic radiation irradiation device 16 centering around the pan axis 21 under the control of the radiotherapy apparatus controller 2. The tilt axis driving device rotates the intermediate member 82 centering around the tilt axis 22 under the control of the radiotherapy apparatus controller 2.

The head swing mechanism 15 further supports the therapeutic radiation irradiation device 16 so that an intersection of the pan axis 21 and the tilt axis 22 conforms with the hypothetical point radiation source 58 of the therapeutic radiation irradiation device 16. In such radiotherapy apparatus 3, when the couch 41, the O-ring 12, and the traveling gantry 14 are secured, the hypothetical point radiation source 58 of the therapeutic radiation irradiation device 16 is secured against the patient 43. In this case, when radiating the therapeutic radiation 23 respectively to a plurality of areas of the patient 43 by moving the therapeutic radiation irradiation device 16 using the head swing mechanism 15, the radiotherapy apparatus 3 can radiate the therapeutic radiation 23 from the same point to a plurality of the areas, respectively.

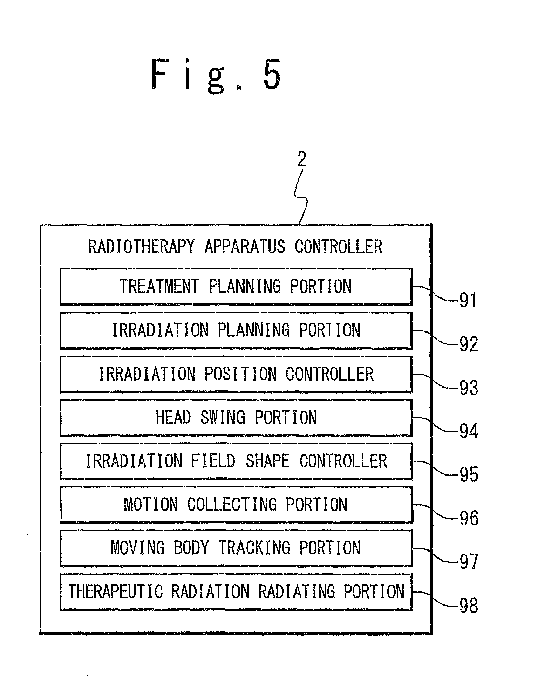

Fig. 5 shows the radiotherapy apparatus controller 2. The radiotherapy apparatus controller 2 is a computer, and includes a CPU, a storage device, an input device, an output device, and an interface that are not shown in Fig. 5. The CPU executes computer programs installed in the radiotherapy apparatus controller 2, and controls the storage device, the input device, the output device, and the interface. The storage device stores the computer programs, stores information used by the CPU, and stores information created by the CPU. The input device supplies information created by a user's operation to the CPU. As the input device, a keyboard and a mouse are shown as examples. The output device outputs the information created by the CPU so as to be recognized by the user. As the output device, a display for showing images created by the CPU is shown as an example. The interface outputs information created by an outside device connected with the radiotherapy apparatus controller 2 to the CPU, and outputs information created by the CPU to the outsidedevice. The outside device includes the radiotherapy apparatus 3.

The radiotherapy apparatus controller 2 includes a treatment planning portion 91, an irradiation planning portion 92, an irradiation position controller 93, an head swing portion 94, an irradiation field shape controller 95, a motion collecting portion 96, a moving body tracking portion 97, and a therapeutic radiation radiating portion 98.

The treatment planning portion 91 shows three dimensional data, which are created by a computer tomography apparatus, indicating a positional relation between an affected area of the patient 43 and internal organs (including a risk part) around the affected area so as to be browsed by a user. The treatment planning portion 91 further creates a treatment plan on the basis of the three dimensional data and information supplied by using the input device. The treatment plan shows three dimensional data of the affected area of the patient 43, and shows a combination of an irradiation angle and a radiation dose. The irradiation angle shows a direction of radiating the therapeutic radiation 23 to the affected area of the patient 43 (that is, a relative position of the patient 43 and the hypothetical point radiation source 58 of the therapeutic radiation irradiation device 16), and shows a traveling angle and a rotational angle. The traveling angle shows a direction of the traveling gantry 14 when the traveling gantry 14 is rotated by the traveling driving device. The rotational angle shows a direction of the O-ring 12 when the O-ring 12 is rotated by the rotation drive device 11. The radiation dose shows a dose of the therapeutic radiation 23 radiated to an affected area from the respective irradiation angles.

The irradiation planning portion 92 creates an irradiation plan for each of combinations of the irradiation and the radiation dose angle shown by the treatment plan created by the treatment planning portion 91. The irradiation plan shows three dimensional data of a plurality of areas obtained by dividing the affected area of the patient 43 by planes including the hypothetical point radiation source 58, and shows combinations of a head swing angle, a shape of an irradiation field, and a radiation dose for each of the plurality of areas. The head swing angle shows a direction of the therapeutic radiation irradiation device 16 when the area is irradiated with the therapeutic radiation 23, and shows a pan angle and a tilt angle. The pan angle shows a direction of the therapeutic radiation irradiation device 16 when the therapeutic radiation irradiation device 16 rotates centering around the pan axis 21 against the intermediate member 82. The tilt angle shows a direction of the intermediate member 82 when the intermediate member 82 rotates centering around the tilt axis 22 against the radiation device support member 81. The shape of an irradiation field shows a sectional shape of the therapeutic radiation 23 radiated to the area. The radiation dose shows a dose of the therapeutic radiation 23 radiated to the area.

The irradiation position controller 93 moves the couch 41 by using the couch driving device 42, and moves the therapeutic radiation irradiation device 16 by using the rotation driving device 11 or the traveling driving device so that a relative position of the patient 43 and the hypothetic point source 58 of the therapeutic radiation irradiation device 16 can meet the irradiation angle shown by the treatment plan created by the treatment planning portion 91.

The head swing portion 94 moves the therapeutic radiation irradiation device 16 by using the head swing mechanism 15 so that a relative position of the patient 43 and the therapeutic radiation irradiation device 16 can meet an irradiation angle shown by an irradiation plan created by the irradiation planning portion 92.

The irradiation field shape controller 95 controls the multi-leaf collimator 56 so that a sectional shape of the therapeutic radiation 23 can meet a shape of an irradiation field shown by an irradiation plan created by the irradiation planning portion 92.

The motion collecting portion 96 detects a motion of the patient 43 by using the imager system of the radiotherapy apparatus 3. That is to say, the motion collecting portion 96 radiates the diagnostic X-ray 35 by using the diagnostic X-ray source 24, and takes a transfer image of the patient 43 produced by using the sensor array 32 on the basis of the diagnostic X-ray 35. Further, the motion collecting portion 96 radiates the diagnostic X-ray 36 by using the diagnostic X-ray source 25, and takes a transfer image of the patient 43 produced by using the sensor array 33 on the basis of the diagnostic X-ray 36. Further, the motion collecting portion 96 radiates the therapeutic radiation 23 by using the therapeutic radiation irradiation device 16, and takes a transfer image of the patient 43 produced by using the sensor array 31 on the basis of the therapeutic radiation 23. The motion collecting portion 96 calculates a marker position by using a plurality of the transfer images taken in such manner. The marker position shows a value indicating a position of a landmark inside the patient 43 in the image when the patient 43 is imaged by the imager system, and shows a motion of the patient 43 . As the landmark, the affected area of the patient 43, a bone (a rib) of the patient 43, a diaphragm, a bladder, and an object embedded in the body of the patient 43 so as to move with the affected area are shown as examples. The object is something to be detected by the imager system and a spherical gold marker made of gold is shown as an example.

The moving body tracking portion 97 stores a table for relating a motion detected by the motion collecting portion 96 to a position of the affected area in the patient 43 . The moving body tracking portion 97 refers to the table, and calculates the position of the affected area relating to the motion detected by the motion collecting portion 96. The moving body tracking portion 97 moves the therapeutic radiation irradiation device 16 by using the head swing mechanism 15 so that the position of the affected area can be irradiated with the therapeutic radiation 23.

The therapeutic radiation radiating portion 98 radiates the therapeutic radiation 23 to the position of the affected area by using the therapeutic radiation irradiation device 16 after the therapeutic radiation irradiation device 16 is moved by the moving body tracking portion 97.

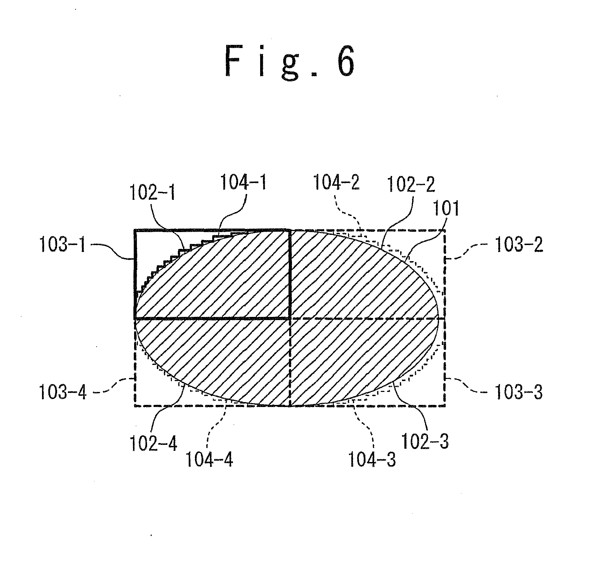

Fig. 6 shows the affected area of the patient 43. The affected area 101 is divided in a plurality of parts 102-1 to 102-4 by planes including the hypothetic point source 58. The part 102-i (i = 1, 2, 3, and 4) is included in a patch area 103-i. A plurality of the patch areas 103-1 to 103-4 respectively shows areas which are irradiated with thetherapeuticradiation23whenthemulti-leafcollimator 56 controls a shape of the irradiation field so that a sectional shape of the therapeutic radiation 23 can be maximum. The plurality of the patch areas 103-1 to 103-4 does not overlap each other. The patch area 103-i includes an irradiation field 104-i. The irradiation field 104-i shows a sectional shape of the therapeutic radiation 23. The irradiation field 104-i is controlled so that the whole part 102-i can be irradiated with the therapeutic radiation 23 and a part irradiated with the therapeutic radiation 23 other than the part 102-i can be as small as possible.

Respective parts 102-i are not necessarily irradiated at same time and in the same shape. For example, in the same head swing angle, each part 102-i may be composed of a combination of a plurality of minute parts which are formed by controlling the shape of the multi-leaf collimator 56. In addition, an irradiation time may be changed for every irradiation of respective minute parts at that time. In such case, this embodiment can be applied to an intensity-modulated irradiation. Only a case of collectively and homogeneously irradiating of respective parts 102-i in a specific shape is shown to simply explain below.

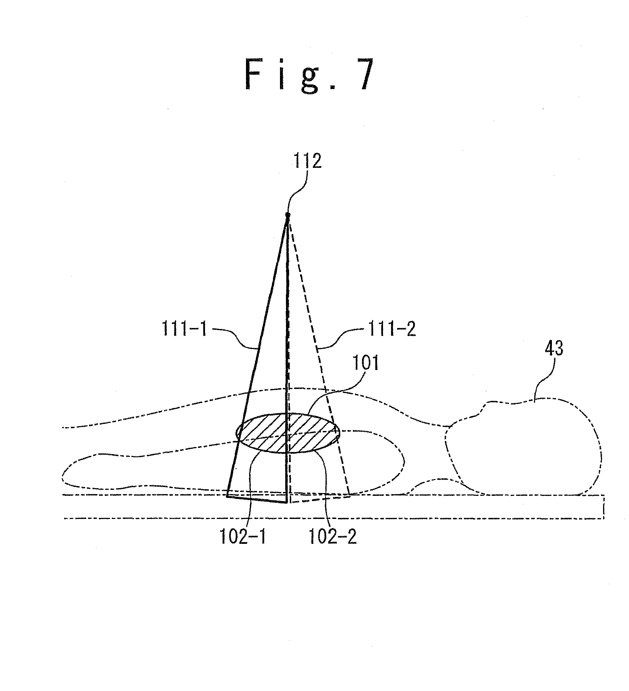

Fig. 7 shows parts irradiated with the therapeutic radiation 23, when a plurality of the parts 102-1 to 102-2 are irradiated with the therapeutic radiation 23. The area 111-1 shows an area irradiated with the therapeutic radiation 23 with which the part 102-1 is irradiated. The area 111-2 shows an area irradiated with the therapeutic radiation 23 with which the part 102-2 is irradiated. The area 111-1 includes an irradiation field 104-1, and is formed in a corn shape whose apex is arranged at a position 112. The position of the apex meets a position of the hypothetic point source 58 that is made when the therapeutic radiation irradiation device 16 radiates the therapeutic radiation 23 to the part 102-1. The area 111-2 includes an irradiation field 104-2, and is formed in a cone shape whose apex is arranged at the position 112. The position of the apex meets a position of the hypothetic point source 58 that is made when the therapeutic radiation irradiation device 16 radiates the therapeutic radiation 23 to the part 102-2. That is to say, the apex of the cone of the area 111-1 and the apex of the cone of the area 111-2 meet at the position 112, and positions of the hypothetic point source 58 meet at the same point when the therapeutic radiation irradiation device 16 radiates the therapeutic radiation 23 to the part 102-1 and when the therapeutic radiation irradiation device 16 radiates the therapeutic radiation 23 to the part 102-2.

In this time, for the purpose of reducing radiation dose to healthy tissue, it is preferable that there is no overlapping of the area 111-1 and the area 111-2. However, in the case of applying the aforementioned intensity-modulated irradiation, it is permissible to have the overlapping within a degree determined by a treatment plan created by the irradiation planning portion 92.

As a result, for the purpose of making possible to control the overlapping, it is preferable that the apex of the cone meets to the hypothetic point source 58. In order to do this, it is preferable to be configured so that the intersection of the pan axis 21 and the tilt axis 22 can meet to the hypothetic point source 58.

That is, the irradiation planning portion 92 divides the affected area 101 into a plurality of the parts 102-1 to 102-4 for every combination of an irradiation angle and a radiation dose shown by a treatment plan created by the treatment planning portion 91, calculates the patch area 103-i including the part 102-i, and calculates an head swing angle when the therapeutic radiation irradiation device 16 turns on the patch area 103-i. The irradiation planning portion 92 further calculates a shape of the irradiation field 104-i so that the whole part 102-i can be irradiated with the therapeutic radiation 23 and a part irradiated with the therapeutic radiation 23 other than the part 102-i can be as small as possible.

The embodiment of the radiation irradiation method according to the present invention is carried out by using the radiotherapy system 1. A user creates a treatment plan by using the radiotherapy apparatus controller 2 at first. The treatment plan shows an irradiation angle in irradiating the affected area of the patient 43 with the therapeutic radiation 23, and shows a radiation dose and a property of the therapeutic radiation 23 radiated from the respective irradiation angles. Next, the radiotherapy apparatus controller 2 creates irradiation plans for every combination of the irradiation angle and the radiation dose shown by the treatment plan. The irradiation plan shows three dimensional data of a plurality of parts formed by dividing the affected area of the patient 43 by a plane including the hypothetic point source 58, and shows combinations of an head swing angle, a shape of the irradiation field and a radiation dose for each of the plurality of the parts. The head swing angle shows a direction of the therapeutic radiation irradiation device 16 when the parts are irradiated with the therapeutic radiation 23, and shows a pan angle and a tilt angle. The shape of the irradiation field shows a sectional shape of the therapeutic radiation 23 with which the parts are irradiated. The radiation dose shows a dose of the therapeutic radiation 23 with which the parts are irradiated, and meets a radiation dose shown by the treatment plan.

A user secures the patient 43 to the couch 41 of the radiotherapy apparatus 3. The radiotherapy apparatus controller 2 moves the couch 41 by using the couch driving device 42 so that the affected area 101 of the patient 43 can be irradiated with the therapeutic radiation 23 at the irradiation angle shown by the treatment plan, rotates the O-ring 12 by using the rotation driving device 11, and rotates the traveling gantry 14 by using the traveling driving device. The radiotherapy apparatus controller 2 moves the therapeutic radiation irradiation device 16 by using the head swing mechanism 15 so that the part 102-i in the affected area 101 of the patient 43 can be irradiated with the therapeutic radiation 23.

In addition, the radiotherapy apparatus controller 2 is also able to irradiate the respective parts 102-i in the affected area 101 of the patient 43 with the therapeutic radiation 23 without using the head swing mechanism 15. For example, the radiotherapy apparatus controller 2 controls an irradiation field by using the multi-leaf collimator 56 so that the part 102-i in the affected area 101 of the patient 43 can be irradiated with the therapeutic radiation 23 after moving the therapeutic radiation irradiation device 16 by using the head swing mechanism 15 so that the therapeutic radiation irradiation device 16 can face the affected area 101 of the patient 43.

Subsequently, the radiotherapy device controller 2 repeats a tracking operation and an irradiation operation for every part 102-i. In the tracking operation, the radiotherapy apparatus controller 2 calculates a position of an affected area on the basis of a position of a landmark detected by the imager system of the radiotherapy apparatus 3. The radiotherapy apparatus controller 2 moves the therapeutic radiation irradiation device 16 by using the head swing mechanism 15 so that the therapeutic radiation 23 can penetrate the position of the affected area. In the irradiation operation, the radiotherapy apparatus controller 2 irradiates the affected area with the therapeutic radiation 23 by using the therapeutic radiation irradiation device 16 just after the therapeutic radiation irradiation device 16 is moved in the tracking operation. Here, an evaluation object of the position of the affected area may be the affected area 101, and may be a center of each part 102-i. However, since the shape of the irradiation field is controlled for each part 102-i, it is preferable to set each part 102-i as an evaluation obj ect .

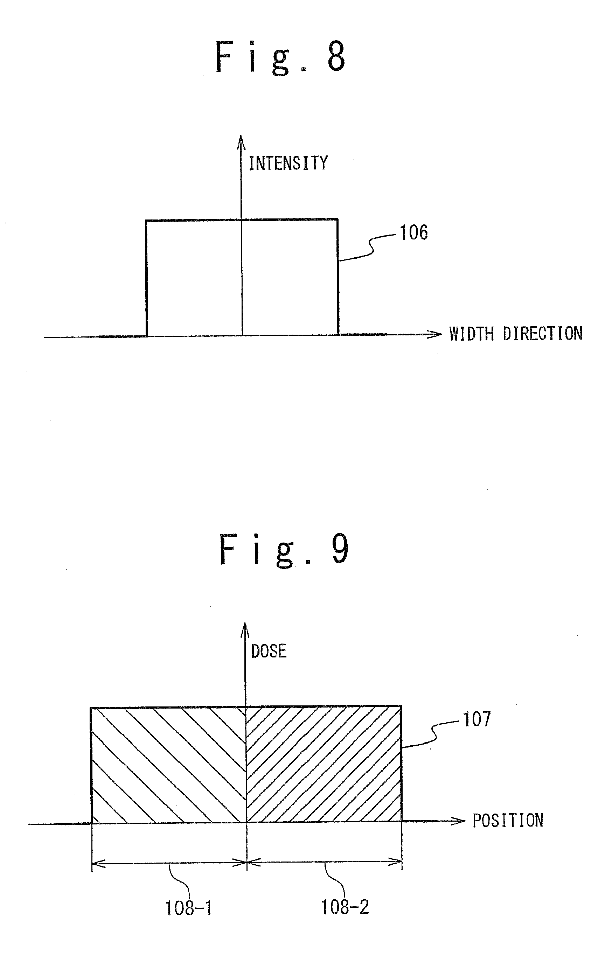

Fig. 8 shows an intensity distribution of the therapeutic radiation 23 with which a predetermined plane is irradiated. The plane is perpendicular to a radiation direction of the therapeutic radiation 23, and is at the position equivalent to the depth of 10 cm in water. The distribution 106 shows that, with respect to a width direction that is perpendicular to the radiation direction, intensity within an irradiation range included in a part irradiated with the therapeutic radiation 23 is almost constant. The distribution 106 further shows that intensity in a non-irradiation range except the irradiation range is sufficiently low in comparison with intensity within the irradiation range. In Japan, the intensity in the non-irradiation range is regulated so as to be equal to or less than one-thousandth of the intensity within the irradiation range by the Enforcement Regulations of Medical Law.

Fig. 9 shows a distribution of dose of the therapeutic radiation 23 with which the plurality of the parts 102-1 to 102-2 are irradiated with executing the radiation irradiation method according to the present invention. The distribution 107 shows a dose of the therapeutic radiation 23 with which a minute part on a plane is irradiated, when the therapeutic radiation 23 with the intensity of the distribution 106 is used, in a case where there is no overlapping area between the area 111-1 and the area 111-2. The plane is perpendicular to the radiation direction of the therapeutic radiation 23 in the plurality of the parts 102-1 to 102-2, and is at the position equivalent to the depth of 10 cm in water. The distribution 107 shows that, with respect to the width direction that is perpendicular to the radiation direction, a radiation dose is almost constant value in a range 108-1 corresponding to the part 102-1, and shows that a radiation dose is almost constant value in a range 108-2 corresponding to the part 102-2. The distribution 107 further shows that the radiation dose in the range 108-1 is almost equal to that in the range 108-2. The distribution 107 further shows that a radiation dose in a range except for the range 108-1 and the range 108-2 is sufficiently smaller than those in the range 108-1 and the range 108-2. According to the present invention, a distribution of a dose of the therapeutic radiation 23 with which a minute part on a plane differently positioned to the radiation direction is similar to the distribution 107, and a dose of the therapeutic radiation 23 with which a plurality of the parts 102-1 to 102-4 is irradiated can be controlled in a similar way of other radiation irradiation apparatus irradiating a plurality of the parts 102-1 to 102-2 at a time.

Incidentally, Fig. 9 is explained regarding a simply explained case where the distribution of the dose of the therapeutic radiation 23 is in a top-flatted shape as shown in Fig. 8. However, since this flatted part is in the case that the position equivalent to the depth of 10 cm in water when the pan angle and the tilt angle is adjusted at 0 degrees, and a flatted part will be generally different from this part in a case of other head swing angle and depth. The greater a difference between the head swing angle and the depth becomes, the greater an extent of the difference becomes. For this reason, its influence is not a problem when a dimension of the depth direction in the affected area 101 is sufficiently small.

In addition, an irradiation condition of a treatment plan is set including this influence. For this reason, when such influence is noticeable depending on a property of an affected area (for example, when a dimension of a depth direction in an affected area is large), it is especially effective to execute aforementioned intensity-modulated irradiation. In addition, since the present invention is not limited to only a case where a distribution of a radiation dose in a specific position becomes top-flatted shape by using the flattening filter 54, it is possible, by optimizing an irradiation condition in a similar treatment plan, to address a case where a distribution of a radiation dose accordingly shows the Gaussian distribution without using the flattening filter 54.

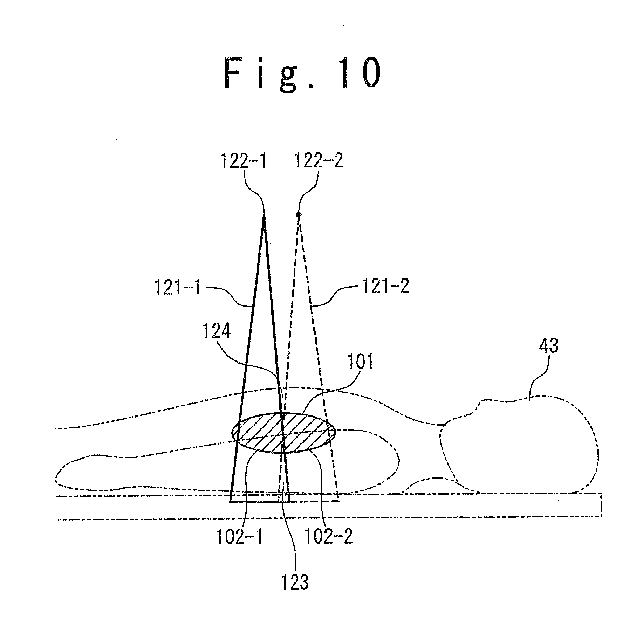

Fig. 10 shows a comparative example of an area irradiated with the therapeutic radiation 23 when a plurality of the parts 102-1 to 102-2 is irradiated with the therapeutic radiation 23. The area 121-1 shows an area irradiated with the therapeutic radiation 23 radiated to the part 102-1. The area 121-2 shows an area irradiated with the therapeutic radiation 23 radiated to the part 102-2. The area 121-1 includes the irradiation field 104-1, and formed in a cone shape whose apex is arranged at a position 122-1. The area 121-2 includes the irradiation field 104-2, and formed in a cone shape whose apex is arranged at a position 122-2. The position 122-2 is different from the position 122-1. At this time, in the affected area of the patient 43, an area 123 that is included in both of the area 121-1 and the area 121-2 exists and an area 124 that is not included in both of the area 121-1 and the area 121-2 exists. That is to say, a distribution of the therapeutic radiation 23 with which a plane perpendicular to its radiation direction is irradiated varies depending on a distance from the position 122-1 (or the position 122-2) of the plane.

That is to say, according to the radiation irradiation method of the present invention, by radiating every part of the affected area of the patient 43 respectively, a predetermined dose of the therapeutic radiation 23 can be radiated to the affected area of the patient 43 with high precision, similar to the case that whole of the affected area of the patient 43 is irradiated at a time. For this reason, since the therapeutic radiation irradiation device 16 is not required to radiate the therapeutic radiation 23 to the whole of the affected area of the patient 43, the therapeutic radiation irradiation device 16 can be designed for radiating the therapeutic radiation 23 only to narrower range, and dimensions of the multi-leaf collimator 56 can be minimized. As a result, since the deflection of the traveling gantry 12 caused by weight of the therapeutic radiation irradiation device 16 decreases, the therapeutic radiation irradiation device 16 is positioned with higher-precision than ever and can radiate the therapeutic radiation 23 to the affected area of the patient 43 with higher-precision than ever. Further, at this time, the multi-leaf collimator 56 can control the irradiation field of the therapeutic radiation 23 with higher-precision than ever, since the plurality of the leaves for shielding against a part of the radiation 60 can be designed so as to be small and the deflection of the leaf caused by its own weight is avoided. The multi-leaf collimator 56 can be further designed so that a thickness of the leaf can be thin more than ever, and resolution of the irradiation field of the therapeutic radiation 23 can be further improved at this time.

A volume of the hypothetic point source 58 is not generally 0, and indicates a positive quantity. The radiation irradiation method of the present invention respectively radiates the therapeutic radiation 23 to a plurality of parts of an affected area of the patient 43 by moving the therapeutic radiation irradiation device 16 so that a point included in an area occupied by the hypothetic point source 58 cannot move against the patient 43. According to such radiation irradiation method, since a part irradiated with the therapeutic radiation 23 more than once and a part that is not irradiated with the therapeutic radiation 23 in the affected area of the patient 43 are sufficiently small when the volume of the hypothetic point source 58 is sufficiently small, a predetermined dose of the therapeutic radiation 23 can be radiated to the affected area of the patient 43 with high precision in a similar way of irradiating the whole of the affected area of the patient 43 at a time.

In addition, the radiation irradiation method of the present invention can be carried out even when other radiotherapy apparatus different from the radiotherapy apparatus 3 in the above-mentioned embodiment is used. In the radiotherapy apparatus, the therapeutic radiation irradiation device 16 in the above-mentioned embodiment is supported so as to be able to move by other driving mechanism. As the driving mechanism, a robot arm is shown as an example. In the radiation irradiation method of the present invention, the therapeutic radiation 23 is radiated respectively to the plurality of the parts 102-1 to 102-4 of the affected area 101 when the plurality of the parts 102-1 to 102-4 is respectively irradiated so that a relative position of the hypothetic point source 58 of the therapeutic radiation irradiation device 16 against the patient 43 cannot change when the plurality of the parts 102-1 to 102-4 of the affected part 101 is respectively irradiated. According to such radiation irradiation method, the therapeutic radiation 23 can be radiated to the affected area of the patient 43 with higher precision than ever, dimensions of the multi-leaf collimator 56 can be minimized, and a resolution of the therapeutic radiation 23 in the irradiation field can be improved in a similar way of the radiation irradiation method in the above-mentioned embodiment.

In addition, the radiation irradiation method of the present invention can abbreviate the tracking operation. At this time, the radiotherapy apparatus controller 2 moves the therapeutic radiation irradiation device 16 by using the head swing mechanism 15 so that the therapeutic radiation irradiation device 16 can face the part 102-i of the affected part 101 in the patient 43, and radiates only a predetermined dose of the therapeutic radiation 23 to the part 102-i by using the therapeutic radiation irradiation device 16 after controlling the irradiation field of the therapeutic radiation 23 by using the multi-leaf collimator 56.

According to such radiation irradiation method, the therapeutic radiation 23 can be radiated to the affected area of the patient 43 with higher precision than ever, dimensions of the multi-leaf collimator 56 can be minimized, and a resolution of the therapeutic radiation in the irradiation field can be further improved in a similar way of the radiation irradiation method in the above-mentioned embodiment. Further, such radiation irradiation method is desirable because it is not required for the imager system to be frequently operated for tracking.

In addition, when the therapeutic radiation 23 is radiated to the plurality of the parts 102-1 to 102-2, the radiation irradiation method of the present invention also can radiate the therapeutic radiation 23 so that an overlapping in an area irradiated with the radiation 23 canexist. According to such radiation irradiation method, a situation that the therapeutic radiation 23 is not radiated to a part between the part 102-1 and the part 102-2 certainly can be avoided, or a dose of the therapeutic radiation 23 larger than ever can be radiated to a desired part of the affected area 101.

In addition, the radiation irradiation method of the present invention can radiate the therapeutic radiation 23 so that doses of the therapeutic radiation 23 radiated to the plurality of the parts 102-1 to 102-2 can be different from each other. Such radiation can be operated by changing irradiation times for the plurality of the parts 102-1 to 102-n, respectively, and by changing radiation intensities for the plurality of the parts 102-1 to 102-n, respectively, when the therapeutic radiation 23 is radiated. According to such radiation irradiation method, dose of the therapeutic radiation 23 larger than ever can be radiated to a desired part of the affected area 101.

On equal terms with other radiation irradiation device which irradiates an irradiation area including a plurality of parts at one time, the radiation irradiation method and the radiotherapy apparatus controller according to the present invention can control the radiation dose with which the plurality of the parts is irradiated, by irradiating the plurality of parts of the subject with radiation radiated from the same one point. Further, the radiation irradiation method and the radiotherapy apparatus controller according to the present invention can design the radiation irradiation device in more miniature size in comparison with other radiation irradiation device which irradiates the irradiation area including the plurality of the parts at one time. For this reason, in a support member for supporting the radiation irradiation device, deflection of the support member caused by weight of the radiation irradiation device is reduced, the radiation irradiation device is positioned with higher precision than ever, and a position of a part irradiated with radiation in the subject can be controlled with higher precision than ever.

It is apparent that the present invention is not limited to the above embodiment that may be modified and changed without departing from the scope and spirit of the invention.

## Claims

1. A radiation irradiation method comprising:
(a) irradiating a first part of a subject with first radiation radiated in a radial pattern from a first point; and
(b) irradiating a second part different from said first part of said subject with second radiation radiated in a radial pattern from a second point,
wherein a first position of said first point relative to said subject correspond to a second position of said second point relative to said subject.

2. The radiation irradiation method according to claim 1, wherein said step (a) includes:
(a1) irradiating said first part with said first radiation radiated by a radiation irradiation apparatus (16) which radiates radiation in a radial pattern from a point,
said step (b) includes:
(b1) moving said radiation irradiation apparatus (16) after said step (a1) such that said radiation irradiation apparatus (16) radiates said second radiation.

3. The radiation irradiation method according to claim 1, wherein said step (a) includes:
(a1) irradiating said first part with said first radiation radiated by a radiation irradiation apparatus (16) which radiates radiation in a radial pattern from a point,
said step (b) includes:
(b1) moving said radiation irradiation apparatus (16) after said step (a1) such that said radiation irradiation apparatus (16) radiates said second radiation and said point does not move relatively to said subject, and
(b2) irradiating a third part of said subject with third radiation radiated by said radiation irradiation apparatus (16) while said radiation irradiation apparatus (16) is moving.

4. The radiation irradiation method according to claim 2 or 3, further comprising:
(c) changing a shape of irradiation field irradiated with said radiation in said subject by an irradiation field shape controlling device which shields against a part of said radiation.

5. The radiation irradiation method according to claim 1, further comprising:
(d) detecting a motion of said subject; and
(e) moving a radiation irradiation apparatus (16) which radiates radiation in a radial pattern from a point based on said motion such that said radiation irradiation apparatus (16) radiates said first radiation to said first part and said second radiation to said second part even though said first part and said second part move.

6. A Computer program product with program code means for carrying out all steps according to any of claims 1 to 5 if the program runs on a computer.

7. A Computer program product with program code means according to claim 6 which are stored on a storage means which can be read by the computer.

8. A radiotherapy apparatus controller comprising:
an irradiation portion (98) configured to irradiate a part of a subject with radiation by a radiation irradiation apparatus (16) which radiates said radiation in a radial pattern from a point; and
a head swing portion (94) configured to move said radiation irradiation apparatus (16) by a driving apparatus (15) which moves said radiation irradiation apparatus (16) relatively to said subject such that said point does not move relatively to said subject.

9. The radiotherapy apparatus controller according to claim 8, further comprising:
a irradiation field shape controller (95) configured to change a shape of irradiation field irradiated with said radiation in said subject by an irradiation field shape controlling device (56) which shields against a part of said radiation.

10. The radiotherapy apparatus controller according to claim 8 or 9, further comprising:
a motion collecting portion (96) configured to collect a motion of said subject by a motion detecting apparatus (16,24,25,31,32,33) which detects said motion; and
a moving body tracking portion (97) configured to move said radiation irradiation apparatus (16) based on said motion by said driving apparatus (15) such that said radiation irradiation apparatus (16) radiates said radiation to said part even though said part moves.

11. A radiotherapy apparatus comprising:
a support member (81) configured to be movable to a subject;
a radiation irradiation apparatus (16) configured to radiate radiation from a point; and
a head swing mechanism (94) configured to support said radiation irradiation apparatus (16) movable to said support member (81) such that said point is fixed to said support member (81).

12. The radiotherapy apparatus according to claim 11, wherein said head swing mechanism (94) supports said radiation irradiation apparatus (16) to said support member (81) such that said radiation irradiation apparatus (16) is rotatable to rotational axes.

13. The radiotherapy apparatus according to claim 11, wherein said head swing mechanism includes:
an intermediate member (82) which is supported by said support member (81) such that said intermediate member (82) is rotatable centering around a first axis,
wherein said radiation irradiation apparatus (16) is supported by said intermediate member (82) such that said radiation irradiation apparatus (16) is rotatable centering around a second axis, and
said first axis intersects with said second axis.

14. A radiotherapy system comprising:
a radiation irradiation apparatus (16) configured to radiate radiation in a radial pattern from a point and have a driving apparatus (15) which moves said radiation irradiation apparatus (16) relatively to a subject; and
a radiotherapy apparatus controller (2) according to any of claims 8 to 10, configured to control said radiation irradiation apparatus (16) and said driving apparatus (15) .
